# EUROPEAN PATENT SPECIFICATION

(11) **EP 1 332 225 B1**
(45) Date of publication and mention of the grant of the patent: **05.11.2008**
(21) Application number: 01975852.3
(22) Date of filing: 11.10.2001
(51) Int. Cl.: C12Q 1/68, G01N 33/08

(54) **METHOD OF IDENTIFYING ABNORMAL IMMUNE REACTIONS**
VERFAHREN ZUR IDENTIFIZIERUNG ABNORMALER IMMUNREAKTIONEN
METHODE POUR IDENTIFIER DES REACTIONS IMMUNITAIRES ANORMALES

(30) Priority: 13.10.2000 AU PR077900
(43) Date of publication of application: 06.08.2003
(73) Proprietor: Advanced Diagnostic Systems Pty Ltd., Subiaco, Western Australia (AU)
(72) Inventor: HOLT, Patrick, Nedlands, Western Australia 6009 (AU); MACAUBAS, Claudia, Menlo Park, CA 94025 (US)
(74) Representative: Miles, John Stephen
(86) International application number: PCT/AU2001/001282
(87) International publication number: WO 2002/031185

(56) References cited:
- WO-A-99/31504
- WO-A-99/49083
- ROMAGNANI S: "Atopic allergy and other hypersensitivities interactions between genetic susceptibility, innocuous and/or microbial antigens and the immune system" CURRENT OPINION IN IMMUNOLOGY, CURRENT BIOLOGY LTD, vol. 9, no. 6, December 1997 (1997-12), pages 773-775, XP004331449 ISSN: 0952-7915
- SRIVASTAVA M D: "IMMUNOMODULATORY EFFECTS OF ETANERCEPT (TNFR:FC) AND ITS USE IN A PATIENT WITH CROHN'S DISEASE" RESEARCH COMMUNICATIONS IN MOLECULAR PATHOLOGY AND PHARMACOLOGY, PJD PUBLICATIONS, WESTBURY, NY, US, vol. 109, no. 1/2, 2001, pages 125-141, XP009040370 ISSN: 1078-0297
- C. MACAUBAS AND P.G. HOLT: 'Regulation of cytokine production in T-cell response to inhalant allergen GATA-3 expression distinguishes between Th1- and Th2-polarised immunity' INTERNATIONAL ARCHIVES OF ALLERGY AND IMMUNOLOGY vol. 124, 2001, pages 176 - 179, XP002986638
- Y. NAKAMURA ET AL.: 'Gene expression of the GATA-3 transcription factor is increased in atopic asthma' THE JOURNAL OF ALLERGY AND CLINICAL IMMUNOLOGY vol. 103, no. 2 PT 1, 1999, pages 215 - 222, XP002986639
- Y. NAKAMURA ET AL.: 'Upregulation of the transcription factor GATA-3 in upper airway mocusa after in vivo and in vitro allergen challenge' THE JOURNAL OF ALLERGY AND CLINICAL IMMUNOLOGY vol. 105, no. 6 PT 1, 2000, pages 1146 - 1152, XP009056112
- O. GHAFFAR ET AL.: 'In vivo expression of signal transducer and activator of transcription factor 6 (STAT6) in nasal mucosa from atopic allergic rhinitis: effect of topical corticosteroids' CLINICAL AND EXPERIMENTAL ALLERGY vol. 30, no. 1, 2000, pages 86 - 93, XP002985197
- J. WALKER AND K. RIGLEY: 'Gene expression profiling in human peripheral blood mononuclear cells using high-density filter-based cDNA microarrays' JOURNAL OF IMMUMNOLOGICAL METHODS vol. 239, 2000, pages 167 - 179, XP004204326
- A.A. ALIZADEH AND L.M. STAUDT: 'Genomic-scale gene expression profiling of normal and malignant immune cells' CURRENT OPINION IN IMMUNOLOGY vol. 12, 2000, pages 219 - 225, XP004257646
- J. OLLILA AND M. VIHINEN: 'Stimulation of B and T cells activates expression of transcription and differentiation factors' BIOCHEMICAL AND BIOPHYSICAL RESEARCH COMMUNICATIONNS vol. 249, 1998, pages 475 - 480, XP002985198
- G. WIEGAND ET AL.: 'Gene expression pattern in human monocytes as a surrogate marker for systemic inflammatory response syndrome (SIRS)' MOLECULAR MEDICINE vol. 5, no. 3, 1999, pages 192 - 202, XP008037023
- GRIFFITHS A. ET AL.: 'Functional genomics', 1999, W.H. FREEMAN AND COMPANY, NEW YORK, ISBN 0-7167-3597-0 pages 399 - 405, XP001237219

## Description

This invention relates to methods for diagnosis of immunologically-mediated conditions, such as allergic conditions, autoimmune diseases, and immunological deficiency, and of assessing immunological reactivity associated with conditions such as viral infections, cancer, AIDS, tissue transplantation and the like. In one preferred embodiment, the invention relates particularly to the diagnosis of atopic asthma.

### BACKGROUND OF THE INVENTION

It will be clearly understood that, although a number of prior art publications are referred to herein, this reference does not constitute an admission that any of these documents forms part of the common general knowledge in the art.

Transcription factors are proteins which function in the transcriptional regulation of genes (Murphy et al., 2000). Of particular interest in immunology and inflammation are transcription factors involved in the upregulation or downregulation of genes encoding biological response modifiers (BRM) such as cytokines, especially those produced by T-lymphocytes.

Skin prick test and IgE reactivity in response to test antigens are frequently used in the diagnosis of allergy and asthma. It is known that the different patterns of skin prick test and IgE reactivity observed in allergic and non-allergic individuals are due to variations in underlying patterns of stimulus-specific T-lymphocyte cytokine gene expression (Romagnani, 1991), but the inherent complexities in these patterns complicate interpretation of the results of *in vitro* tests involving stimulus-induced cytokine mRNA or cytokine protein (Borish and Rosenwasser, 1997).

Recent research from a number of laboratories has demonstrated that during differentiation of T-lymphocytes following stimulation with agents such as specific antigens or polyclonal mitogens, the genes encoding certain transcription factors, such as GATA-3, t-bet, NFAT, NFKκB, ROG, STAT4, STAT6, IRF-1, and c-maf are themselves transcribed early in the differentiation process, and subsequently exert specific effects upon transcription of cytokine genes in T-lymphocytes (see for example Zheng and Flavell,1997; Ho and Glimcher, 1998; Zhang et al., 1997; Murphy et al., 2000; Miaw et al., 2000).

Current methods available for monitoring this activation/differentiation process in lymphocytes rely upon measurement of either intracellular or secreted BRM as protein, or via detection of specific mRNA molecules encoding the BRM. These methods are complicated and unsatisfactory. The different genes encoding various BRM are transcribed at very different rates, sometimes not until several rounds of cell division have occurred after lymphocyte activation (see for example Bird et al., 1998) Moreover, the patterns of BRM gene expression are frequently very heterogeneous, and do not necessarily provide accurate information concerning the functional phenotype of the cells being tested. Consequently there is a need in the art for simpler, more accurate and more rapid methods of assessing patterns of BRM expression.

We have now surprisingly found that stimulation of peripheral blood mononuclear cells (PBMC) from atopic subjects with a specific allergen results in increased expression of mRNA encoding a transcription factor, while in contrast, PBMCs from non-atopic subjects showed a decrease in expression. We therefore envisage measurement of patterns of stimulus-induced transcription factor expression to provide a more informative method of discriminating between the stimulus-induced responses of PBMC from patients and those of control subjects than is currently available. Thus the invention contemplates monitoring events which occur much earlier during stimulus-induced activation/differentiation, notably transcription of genes encoding individual transcription factors, than is possible to monitor by *in vitro* measurement of stimulus-induced cytokine mRNA or cytokine protein.

### SUMMARY OF THE INVENTION

We have demonstrated that when human PBMC are stimulated *in vitro* with a specific allergen such as House Dust Mite (HDM), the expression of mRNA encoding the transcription factor known as GATA-3 is increased in atopic subjects who are positive responders to HDM as measured by skin prick test reactivity and the presence of serum HDM-specific IgE antibody. In contrast, the expression of this mRNA is decreased in non-atopic subjects who are SPT negative/IgE negative; a similar propensity for GATA-3 expression in some atopics was observed when their PBMC were cultured with a polyclonal mitogen.

Accordingly, the invention generally provides a method of immunodiagnosis, comprising the step of measuring stimulus-induced and resting levels of expression of one or more transcription factors in a population of leukocytes. The stimulus may for example be an antigen.

In a first aspect, the invention provides a method of assessing an immunological reaction in a test subject suspected to be manifesting an abnormal immunological reaction, comprising the steps of
(a) substantially isolating leukocytes from a biological sample taken from a test subject and from a control subject;
(b) stimulating said leukocytes by exposure to a stimulus;
(c) measuring the level of expression of a transcription factor in said leukocytes; and
(d) comparing the levels of expression in stimulated leukocytes in the test subjects and in the control subjects and comparing the levels of expression to unstimulated leukocytes taken from these subjects,
wherein significantly different expression in the test subject than in the control subject is indicative of an abnormal immunological reaction.

It will be appreciated that the test subject may show either higher or lower expression than the control, and both are indicative of abnormal immunological reaction.

The stimulus may be any agent which is capable of inducing expression of a transcription factor which modulates expression of a cytokine, including but not limited to specific allergens, heterologous or self antigens, mitogens such as concanavalin A or phytohaemagglutinin, and hormones such as progesterone, oestrogen or oestradiol. The leukocytes to be tested may be from any biological sample, but are most conveniently PBMC or unfractionated peripheral blood leukocytes or specific subfractions thereof. It will be clearly understood that the use of leukocytes from lymphoid organs or other tissues, including tumours, is also within the scope of the invention.

The transcription factor may be any transcription factor which is elicited in response to a stimulus which acts on the immune system, is transcribed early in the differentiation process, and subsequently exerts specific effects upon transcription of cytokine genes in T-lymphocytes. Such transcription factors include, but are not limited to GATA-3, t-bet, NFAT, NFKκB, ROG, STAT4, STAT6, IRF-1, and c-maf.

Measurement of transcription factor expression may be effected by any convenient method, such as:
(i) detection and quantitation of specific mRNA encoding the transcription factor, by polymerase chain reaction (PCR) or reverse transcriptase-PCR(RT-PCR), real-time PCR, or via specific DNA arrays utilising microchip technology or by slot blot hybridisation;
(ii) measurement of the transcription factor protein by methods such as ELISA, proteomic arrays, intracellular staining as detected by flow cytometry, or by Western blot.
The person skilled in the art will be aware of suitable methods.

Changes in the physiochemical state of transcription factor, including post-translational modifications such as phosphorylation which are involved in activation and signal transduction may also be measured. Suitable methods for doing so are known in the art.

In preferred embodiments, the method of the invention may be used:
(i) as a diagnostic adjunct in detection of immunologically-mediated conditions such as allergy, autoimmunity, graft rejection or immunodeficiency;
(ii) as a diagnostic adjunct in assessment of immune reactivity to antigens associated with conditions such as cancer, viral infection, AIDS, or tissue transplantation;
(iii) to assist in monitoring response of patients to treatments such as immunotherapy or immunosuppression;
(iv) for mass screening for diseases such as tuberculosis, rheumatoid arthritis, or type 1 diabetes;
(v) for assessment of potential disease susceptibility e.g. detection of subjects at high risk of autoimmunity, atopy, or asthma;
(iv) routine assessment of immune competence, by testing transcription factor expression following polyclonal stimulation.

For the purposes of this specification it will be clearly understood that the word "comprising" means "including but not limited to", and that the word "comprises" has a corresponding meaning.

### BRIEF DESCRIPTION OF THE FIGURE

Figure 1 compares the expression of mRNA encoding the transcription factor GATA-3 in PBMC from atopic subjects (HDM IgE positive) and non-atopic subjects (HDM IgE negative) following culture in the presence of HDM allergen or of a polyclonal stimulant, monoclonal antibody directed against CD3.
Figure 2 compares the expression of gata-3 and c-maf in PBMC from a second panel of atopics and non-atopic subjects following culture in the presence of HDM.

### DETAILED DESCRIPTION OF THE INVENTION

The practice of the present invention employs, unless otherwise indicated, conventional molecular biology, cellular biology, and recombinant DNA techniques within the skill of the art. Such techniques are well known to the skilled worker, and are explained fully in the literature. *See,* e.g., Sambrook and Russell "Molecular Cloning: A Laboratory Manual" (2001); Cloning: A Practical Approach," Volumes I and II (D.N. Glover, ed., 1985); "Oligonucleotide Synthesis" (M.J. Gait, ed., 1984); "Nucleic Acid Hybridisation" (B.D. Hames & S.J. Higgins, eds., 1985); "Antibodies: A Laboratory Manual" (Harlow & Lane, eds., 1988); "Transcription and Translation" (B.D. Hames & S.J. Higgins, eds., 1984); "Animal Cell Culture" (R.I. Freshney, ed., 1986); "Immobilised Cells and Enzymes" (IRL Press, 1986); B. Perbal, "A Practical Guide to Molecular Cloning" (1984), and Sambrook, et al., "Molecular Cloning: a Laboratory Manual" (1989). Ausubel, F. et al., 1989-1999, "Current Protocols in Molecular Biology" (Green Publishing, New York).

Before the present methods are described, it is understood that this invention is not limited to the particular materials and methods described, as these may vary. It is also to be understood that the terminology used herein is for the purpose of describing particular embodiments only, and is not intended to limit the scope of the present invention which will be limited only by the appended claims. It must be noted that as used herein and in the appended claims, the singular forms "a," "an," and "the" include plural reference unless the context clearly dictates otherwise. Thus, for example, a reference to "a transcription factor" includes a plurality of such factors, and a reference to "a primer" is a reference to one or more primers and equivalents thereof known to those skilled in the art, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.

All publications mentioned herein are cited for the purpose of describing and disclosing the cell lines, protocols, reagents and vectors which are reported in the publications and which might be used in connection with the invention. Nothing herein is to be construed as an admission that the invention is not entitled to antedate such disclosure by virtue of prior invention.

In describing the present invention, the following terminology is used in accordance with the definitions set out below.

### ABBREVIATIONS USED

- BSA: Bovine serum albumin
- DMSO: Dimethyl sulfoxide
- DNA: Deoxyribonucleic acid
- EDTA: Ethylenediaminetetraacetic acid
- FAM: Amine-reactive succinimidyl ester of carboxyfluorescein
- Flu: Fluorescein
- IL: Interleukin
- LC-640: Lightcycler^{®} Red 640
- LED: Light emitting diode
- PCR: Polymerase chain reaction
- RAST: Radioallergosorbent test
- RNA: Ribonucleic acid
- RT-PCR: Reverse-transcriptase polymerase chain reaction
- Taq: *Thermophilus aquaticus*
- Tₘ: Melting temperature

### DEFINITIONS

"Transcription factor" or "transcription factor protein" refers to a polypeptide or protein encoded by a transcription factor polynucleotide sequence; a polypeptide that is the translated amino acid sequence of a polynucleotide sequence; fragments thereof having greater than about 5 amino acid residues and comprising an immune epitope or other biologically active site of a transcription factor protein.

The terms "nucleic acid" and "polynucleic acid" refer herein to deoxyribonucleic acid and ribonucleic acid in all their forms, i.e., single and double-stranded DNA, cDNA, mRNA, and the like. As used herein, the term "encode" in its various grammatical forms includes nucleotides and/or amino acids that correspond to other nucleotides or amino acids in the transcriptional and/or translational sense.

As such, "cytokine transcription factor nucleic acid" is RNA or DNA that encodes a transcription factor. "Isolated" cytokine transcription factor nucleic acid is cytokine transcription factor nucleic acid that is separated from (or otherwise substantially free from), contaminant nucleic acid encoding other polypeptides. The isolated cytokine transcription factor nucleic acid can be labelled for diagnostic and probe purposes, using a label as described further herein in the discussion of diagnostic assays and nucleic acid hybridization methods.

For example, isolated cytokine transcription factor DNA, or a fragment thereof comprising at least about 15 nucleotides, can be used as a hybridization probe to detect amplified cytokine transcription factor cDNA resulting from increased mRNA expression, such as may result from stimulation of PMBC with specific allergens. In one embodiment of the invention, total RNA in a biological sample from a subject (that is, a human or other mammal) can be assayed for the presence of cytokine transcription factor mRNA, wherein the increase in the amount of cytokine transcription factor mRNA is a result of stimulus-induced activation/differentiation of transcription factors.

One purpose of the present invention is to identify changes in the level of transcription factor nucleic acid expression after exposure to allergens. There are many techniques for detecting nucleic acid expression that can be employed. Cytokine transcription factor expression may be measured in a biological sample directly, for example, by conventional Southern blotting to quantitate DNA, or Northern blotting to quantitate mRNA, using an appropriately labelled oligonucleotide hybridization probe, based on the known sequences of cytokine transcription factor. Identification of cytokine transcription factor mRNA within a mixture of various mRNAs, is conveniently accomplished by the use of reverse transcriptase-polymerase chain reaction and an oligonucleotide hybridization probe that is labelled with a detectable moiety. Various labels may be employed, most commonly radioisotopes, particularly ³²P. However, other techniques may also be employed, such as using biotin-modified nucleotides for introduction into a polynucleotide. The biotin then serves as the site for binding to avidin or antibodies, which may be labelled with a wide variety of labels, such as radioisotopes, fluorophores, chromophores, or the like. Keller, et al., DNA Probes, pp.149-213 (Stockton Press, 1989). Alternatively, antibodies may be employed that can recognise specific duplexes, including DNA duplexes, RNA duplexes, and DNA-RNA hybrid duplexes or DNA-protein duplexes. The antibodies in turn may be labelled and the assay may be carried out where the duplex is bound to a surface, so that upon the formation of duplex on the surface, the presence of antibody bound to the duplex can be detected.

Cytokine transcription gene expression may also be based on the functional or antigenic characteristics of the expressed protein, eg. immunoassays and the like.

In one preferred embodiment, a biological sample is taken from a test subject who is suspected of, of who is susceptible to an abnormal immunological response. Such an individual will likely to be atopic in that they are constitutionally or hereditarily likely to develop immediate hypersensitivity to allergens that provoke no immune reactions in normal subjects. Biological samples may include a sample of tissue or fluid isolated from an individual, including but not limited to bone marrow, plasma, serum, spinal fluid, lymph fluid, the external sections of the skin, respiratory, intestinal, and genitourinary tracts, tears, saliva, milk, blood; both whole blood and anticoagulated whole blood, blood cells, tumours, organs, and also includes samples of in vivo cell culture constituents. However, it is preferable that the biological sample is blood, lymph fluid, or a blood component. Most preferably the biological sample is leukocytes isolated from peripheral blood. Also included in the term are derivatives and fractions of such cells and fluids.

The leukocytes in the biological sample may be substantially isolated ie separated from (or otherwise substantially free from), other contaminant cells. The biological sample is then exposed to an agent which is capable of inducing expression of a transcription factor which modulates expression of a cytokine, including but not limited to specific allergens, heterologous or self antigens, mitogens such as concanavalin A or phytohaemagglutinin, and hormones such as progesterone, oestrogen or oestradiol. This step constitutes the stimulation phase of the described method.

Following exposure to the agent expression levels of cytokine transcription factors are determined or measured.

In one preferred method, mRNA in a biological sample is reverse transcribed to generate a cDNA strand. The cDNA may be amplified by conventional techniques, such as polymerase chain reaction, to provide sufficient amounts for analysis.

Amplification may also be used to determine whether a specific sequence is present, by using a primer that will specifically bind to the desired sequence, where the presence of an amplification product is indicative that a specific binding complex was formed. Alternatively, the mRNA sample is fractionated by electrophoresis, eg. capillary or gel electrophoresis, transferred to a suitable support, eg. nitrocellulose and then probed with a fragment of the transcription factor sequence. Other techniques may also find use, including oligonucleotide ligation assays, binding to solid-state arrays, etc. Detection of mRNA having the subject sequence is indicative gene expression of the transcription factor in the sample.

"Polymerase chain reaction," or "PCR," as used herein generally refers to a method for amplification of a desired nucleotide sequence *in vitro,* as described in U.S. Pat. No. 4,683,195. In general, the PCR method involves repeated cycles of primer extension synthesis, using two oligonucleotide primers capable of hybridizing preferentially to a template nucleic acid. Typically, the primers used in the PCR method will be complementary to nucleotide sequences within the template at both ends of or flanking the nucleotide sequence to be amplified, although primers complementary to the nucleotide sequence to be amplified also may be used. See Wang, et al., in PCR Protocols, pp.70-75 (Academic Press, 1990); Ochman, et al., in PCR Protocols, pp. 219-227; Triglia, et al., Nuc. Acids Res. 16:8186 (1988).

"Oligonucleotides" are short-length, single- or double-stranded polydeoxynucleotides that are chemically synthesized by known methods (involving, for example, triester, phosphoramidite, or phosphonate chemistry), such as described by Engels, et al., Agnew. Chem. Int. Ed. Engl. 28:716-734 (1989). They are then purified, for example, by polyacrylamide gel electrophoresis.

As used herein, the term "PCR reagents" refers to the chemicals, apart from the target nucleic acid sequence, needed to perform the PCR process. These chemicals generally consist of five classes of components: (i) an aqueous buffer, (ii) a water soluble magnesium salt, (iii) at least four deoxyribonucleotide triphosphates (dNTPs), (iv) oligonucleotide primers (normally two primers for each target sequence, the sequences defining the 5' ends of the two complementary strands of the double-stranded target sequence), and (v) a polynucleotide polymerase, preferably a DNA polymerase, more preferably a thermostable DNA polymerase, ie a DNA polymerase which can tolerate temperatures between 90°C and 100°C for a total time of at least 10 minutes without losing more than about half its activity.

The four conventional dNTPs are thymidine triphosphate (dTTP), deoxyadenosine triphosphate (dATP), deoxycitidine triphosphate (dCTP), and deoxyguanosine triphosphate (dGTP). These conventional triphosphates may be supplemented or replaced by dNTPs containing base analogues which Watson-Crick base pair like the conventional four bases, eg deoxyuridine triphosphate (dUTP).

A detectable label may be included in an amplification reaction. Suitable labels include fluorochromes, eg. fluorescein isothiocyanate (FITC), rhodamine, Texas Red, phycoerythrin, allophycocyanin, 6-carboxyfluorexcein (6-FAM), 2',7'-dimethoxy-4',5'-dichloro-6-carboxyfluorescein (JOE), 6-carboxy-X-rhodamine(ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, eg. 32P, 35S, 3H; as well as others. The label may be a two stage system, where the amplified DNA is conjugated to biotin, haptens, or the like having a high affinity binding partner, eg. avidin, specific antibodies, etc., where the binding partner is conjugated to a detectable label. The label may be conjugated to one or both of the primers. Alternatively, the pool of nucleotides used in the amplification is labelled, so as to incorporate the label into the amplification product.

Accordingly, in one preferred embodiment, once the cytokine transcription factor mRNA has been reverse transcribed and amplified by PCR, it is detected by various means including oligonucleotide probes. Oligonucleotide probes of the invention are DNA molecules that are sufficiently complementary to regions of contiguous nucleic acid residues within the cytokine transcription factor nucleic acid to hybridise thereto, preferably under high stringency conditions. Exemplary probes include oligomers that are at least about 15 nucleic acid residues long and that are selected from any 15 or more contiguous residues of DNA of the present invention. Preferably, oligomeric probes used in the practice of the present invention are at least about 20 nucleic acid residues long. The present invention also contemplates oligomeric probes that are 150 nucleic acid residues long or longer. Those of ordinary skill in the art realise that nucleic hybridisation conditions for achieving the hybridisation of a probe of a particular length to polynucleotides of the present invention can readily be determined. Such manipulations to achieve optimal hybridisation conditions for probes of varying lengths are well known in the art. See, e.g., Sambrook et al., Molecular Cloning: A Laboratory Manual, Second Edition, Cold Spring Harbor (1989*)*, incorporated herein by reference.

Preferably, oligomeric probes of the present invention are labelled to render them readily detectable. Detectable labels may be any species or moiety that may be detected either visually or with the aid of an instrument. Commonly used detectable labels are radioactive labels such as, for example, ³²P, ¹⁴C, ¹²⁵I, ³H, and ³⁵S. Examples of fluorescer-quencher pairs may be selected from xanthene dyes, including fluoresceins, and rhodamine dyes. Many suitable forms of these compounds are widely available commercially with substituents on their phenyl moieties which can be used as the site for bonding or as the bonding functionality for attachment to an oligonucleotide. Another group of fluorescent compounds are the naphthylamines, having an amino group in the alpha or beta position. Included among such naphthylamino compounds are 1-dimethylaminonaphthyl-5-sulfonate, 1-anilino-8-naphthalene sulfonate and 2-p-touidinyl-6-naphthalene sulfonate. Other dyes include 3-phenyl-7-isocyanatocoumarin, acridines, such as 9-isothiocyanatoacridine acridine orange; N-(p-(2-benzoaxazolyl)phenyl)maleimide; benzoxadiazoles, stilbenes, pyrenes, and the like. Most preferably, the fluorescent compounds are selected from the group consisting of VIC, carboxy fluorescein (FAM), Lightcycler^{®} 640 and Cy5.

Biotin-labelled nucleotides can be incorporated into DNA or RNA by such techniques as nick translation, chemical and enzymatic means, and the like. The biotinylated probes are detected after hybridisation, using indicating means such as avidin/streptavidin, fluorescent labelling agents, enzymes, colloidal gold conjugates, and the like. Nucleic acids may also be labelled with other fluorescent compounds, with immunodetectable fluorescent derivatives, with biotin analogues, and the like. Nucleic acids may also be labelled by means of attachment to a protein. Nucleic acids cross-linked to radioactive or fluorescent histone single-stranded binding protein may also be used. Those of ordinary skill in the art will recognise that there are other suitable methods for detecting oligomeric probes and other suitable detectable labels that are available for use in the practice of the present invention. Moreover, fluorescent residues can be incorporated into oligonucleotides during chemical synthesis.

Two DNA sequences are "substantially similar when at least about 85%, preferably at least about 90%, and most preferably at least about 95%, of the nucleotides match over the defined length of the DNA sequences. Sequences that are substantially similar can be identified for example in a Southern hybridisation experiment performed under stringent conditions as defined for that particular system. Defining appropriate hybridisation conditions is within the skill of the art. See e.g., Maniatis et al., DNA Cloning, vols. I and II. Nucleic Acid Hybridisation. However, briefly, "stringent conditions" for hybridisation or annealing of nucleic acid molecules are those that (1) employ low ionic strength and high temperature for washing, for example, 0.015M NaCl/0.0015M sodium citrate/0.1% sodium dodecyl sulfate (SDS) at 50°C, or (2) employ during hybridisation a denaturing agent such as formamide, for example, 50% (vol/vol) formamide with 0.1% bovine serum albumin/0.1% Ficoll/0.1% polyvinylpyrrolidone/50mM sodium phosphate buffer at pH 6.5 with 750mM NaCl, 75mM sodium citrate at 42°C. Another example is use of 50% formamide, 5 X SSC (0.75M NaCl, 0.075M sodium citrate), 50mM sodium phosphate (pH 6.8), 0.1% sodium pyrophosphate, 5 X Denhardt's solution, sonicated salmon sperm DNA (50µg/mL), 0.1% SDS, and 10% dextran sulfate at 42°C, with washes at 42°C in 0.2 X SSC and 0.1% SDS.

In a particularly preferred embodiment the present invention utilises a combined PCR and hybridisation probing system so as to make the most of the closed tube or homogenous assay systems such as the use of FRET probes as disclosed in US patents (Nos 6,140,054; 6,174,670), the entirety of which are also incorporated herein by reference. In one of its simplest configurations, the FRET or "fluorescent resonance energy transfer" approach employs two oligonucleotides which bind to adjacent sites on the same strand of the nucleic acid being amplified. One oligonucleotide is labelled with a donor fluorophore which absorbs light at a first wavelength and emits light in response, and the second is labelled with an acceptor fluorophore which is capable of fluorescence in response to the emitted light of the first donor (but not substantially by the light source exciting the first donor, and whose emission can be distinguished from that of the first fluorophore). In this configuration, the second or acceptor fluorophore shows a substantial increase in fluorescence when it is in close proximity to the first or donor fluorophore, such as occurs when the two oligonucleotides come in close proximity when they hybridise to adjacent sites on the nucleic acid being amplified (for example in the annealing phase of PCR) forming a fluorogenic complex. As more of the nucleic acid being amplified accumulates, so more of the fluorogenic complex can be formed and there is an increase in the fluorescence from the acceptor probe, and this can be measured. Hence the method allows detection of the amount of product as it is being formed. In another simple embodiment, and as applies to use of FRET probes in PCR based assays, one of the labelled oligonucleotides may also be a PCR primer used for PCR. In this configuration, the labelled PCR primer is part of the DNA strand to which the second labelled oligonucleotide hybridises, as described by Neoh et al (J Clin Path 1999;52:766-769.), von Ahsen et al (Clin Chem 2000;46:156-161), the entirety of which are encompassed by reference.

It will be appreciated by those of skill in the art that amplification and detection of amplification with hybridisation probes can be conducted in two separate phases-for example by carrying out PCR amplification first, and then adding hybridisation probes under such conditions as to measure the amount of nucleic acid which has been amplified. However, a preferred embodiment of the present invention utilises a combined PCR and hybridisation probing system so as to make the most of the closed tube or homogenous assay systems and is carried out on a Roche Lightcycler^{®} or other similarly specified or appropriately configured instrument.

Such systems would also be adaptable to the detection methods described here. Those skilled in the art will appreciate that such probes can be used for allele discrimination if appropriately designed for the detection of point-mutations, in addition to deletion and insertions. Alternatively or in addition, the unlabelled PCR primers may be designed for allele discrimination by methods well known to those skilled in the art (Ausubel 1989-1999).

It will also be appreciated by those skilled in the art that detection of amplification in homogenous and/or closed tubes can be carried out using numerous means in the art, for example using TaqMan® hybridisation probes in the PCR reaction and measurement of fluorescence specific for the target nucleic acids once sufficient amplification has taken place. However, because of the nature and speed of the Roche Lightcycler^{®}, the preferred method is by using real-time PCR and melting curve analysis on the Roche Lightcycler^{®} using fluorescent labelled hybridisation oligonucleotides.

Although those skilled in the art will be aware that other similar quantitative "real-time" and homogenous nucleic acid amplification/detection systems exist such as those based on the TaqMan approach (US patent Nos 5,538,848 and 5,691,146), fluorescence polarisation assays (eg Gibson et al., Clin Chem, 1997; 43: 1336-1341), and the Invader assay (eg Agarwal P et al., Diagn Mol Pathol 2000 Sep; 9(3): 158-164; Ryan D et al, Mol Diagn 1999 Jun; 4(2): 135-144). Such systems would also be adaptable to use the invention described, enabling real-time monitoring of nucleic acid amplification.

In one embodiment of the present invention an initial procedure involves the manufacture of the oligonucleotide matrices or microchips. The microchips contain a selection of immobilized synthetic oligomers, said oligomers synthesized so as to contain complementary sequences for desired portions of transcription factor DNA. The oligomers are then hybridized with cloned or polymerase chain reaction (PCR) amplified transcription factor nucleic acids, said hybridization occurring under stringent conditions, outlined infra. The high stringency conditions insure that only perfect or near perfect matches between the sequence embedded in the microchip and the target sequence will occur during hybridization.
After each initial hybridization, the chip is washed to remove most mismatched fragments. The reaction mixture is then denatured to remove the bound DNA fragments, which are subsequently labeled with a fluorescent marker. A second round of hybridization with the labeled DNA fragments is then carried out on sequence microchips containing a different set of immobilized oligonucleotides. These fragments first may be cleaved into smaller lengths. The different set of immobilized nucleotides may contain oligonucleotides needed for whole sequencing, partial sequencing, sequencing comparison, or sequence identification. Ultimately, the fluorescence from this second hybridization step can be detected by an epifluorescence microscope coupled to a CCD camera. (See US patent No. 5,851,772 incorporated herein by reference).

Gene expression, alternatively, may be measured by immunological methods, such as immunohistochemical staining of tissue sections and assay of cell culture or body fluids, to quantitate directly the expression of the gene product, cytokine transcription factor. With immunohistochemical staining techniques, a cell sample is prepared, typically by dehydration and fixation, followed by reaction with labelled antibodies specific for the gene product coupled, where the labels are usually visually detectable, such as enzymatic labels, fluorescent labels, luminescent labels, and the like. A particularly sensitive staining technique suitable for use in the present invention is described by Hsu, et al., Am. J. Clin. Path., 75:734-738 (1980). Antibodies useful for immunohistochemical staining and/or assay of sample fluids may be either monoclonal or polyclonal. Conveniently, the antibodies may be prepared against a synthetic peptide based on known DNA sequences of cytokines such as GATA-3, t-bet, NFAT, NFKκB, ROG, STAT4, STAT6, IRF-1, and c-maf.

For example, the cytokine transcription factor peptide may be used as an immunogen to generate anti-cytokine transcription factor antibodies. Such antibodies, which specifically bind to cytokine transcription factor, are useful as standards in assays for cytokine transcription factor, such as by labelling purified cytokine transcription factor for use as a standard in a radioimmunoassay, enzyme-linked immunoassay, or competitive-type receptor binding assays radioreceptor assay, as well as in affinity purification techniques. Ordinarily, the anti-cytokine transcription factor antibody will bind cytokine transcription factor with an affinity of at least about 10⁶ L/mole, and preferably at least about 10⁷ L/mole.

Polyclonal antibodies directed toward cytokine transcription factor generally are raised in animals by multiple subcutaneous or intraperitoneal injections of cytokine transcription factor and an adjuvant. It may be useful to conjugate cytokine transcription factor or a peptide fragment thereof to a carrier protein that is immunogenic in the species to be immunised, such as keyhole limpet haemocyanin, serum albumin, bovine thyroglobulin, or soybean trypsin inhibitor, using a bifunctional or derivatizing agent, for example, maleimidobenzoyl sulfosuccinimide ester (conjugation through cysteine residues), N-hydroxysuccinimide (conjugation through lysine residues), glutaraldehyde, succinic anhydride, SOCl₂, or R¹N = C = NR, where R and R¹ are different alkyl groups.

Animals are immunised with such cytokine transcription factor-carrier protein conjugates combining 1mg or 1µg of conjugate (for rabbits or mice, respectively) with 3 volumes of Freund's complete adjuvant and injecting the solution intradermally at multiple sites. One month later the animals are boosted with 1/5th to 1/10th the original amount of conjugate in Freund's complete adjuvant by subcutaneous injection at multiple sites. 7 to 14 days later animals are bled and the serum is assayed for anti-cytokine transcription factor antibody titre. Animals are boosted until the antibody titre plateaus. Preferably, the animal is boosted by injection with a conjugate of the same cytokine transcription factor with a different carrier protein and/or through a different cross-linking agent. Conjugates of cytokine transcription factor and a suitable carrier protein also can be made in recombinant cell culture as fusion proteins. Also, aggregating agents such as alum are used to enhance the immune response.

Monoclonal antibodies directed toward cytokine transcription factor are produced using any method which provides for the production of antibody molecules by continuous cell lines in culture. The modifier "monoclonal" indicates the character of the antibody as being obtained from a substantially homogeneous population of antibodies, and is not to be construed as requiring production of the antibody by any particular method. Examples of suitable methods for preparing monoclonal antibodies include the original hybridoma method of Kohler, et al., Nature 256:495-497 (1975), and the human B-cell hybridoma method, Kozbor, J. Immunol. 133:3001 (1984); Brodeur, et al., Monoclonal Antibody Production Techniques and Applications, pp.51-63 (Marcel Dekker, Inc., New York, 1987).

For diagnostic applications, anti-cytokine transcription factor antibodies typically will be labelled with a detectable moiety. The detectable moiety can be any one which is capable of producing, either directly or indirectly, a detectable signal. For example, the detectable moiety may be a radioisotope, such as ³H, ¹⁴C, ³²P, ³⁵S, or ¹²⁵I a fluorescent or chemiluminescent compound, such as fluorescein isothiocyanate, rhodamine, or luciferin; radioactive isotopic labels, such as, eg., ¹²⁵I, ³²P, ¹⁴C, or ³H, or an enzyme, such as alkaline phosphatase, beta-galactosidase or horseradish peroxidase.

Any method known in the art for separately conjugating the antibody to the detectable moiety may be employed, including those methods described by David, et al., Biochemistry 13:1014-1021 (1974); Pain, et al., J. Immunol. Meth. 40:219-231 (1981); and Bayer, et al., Meth. Enz. 184:138-163 (1990).

The anti-cytokine transcription factor antibodies may be employed in any known assay method, such as competitive binding assays, direct and indirect sandwich assays, and immunoprecipitation assays. Zola, Monoclonal Antibodies: A Manual of Techniques, pp.147-158 (CRC Press, Inc., 1987). The anti-cytokine transcription factor antibodies may also be used in Western blots performed on protein gels or protein spots on filters, using a detection system specific for the transcription factor as desired, conveniently using a labelling method using conventional techniques.

Competitive binding assays rely on the ability of a labelled standard (eg., cytokine transcription factor or an immunologically reactive portion thereof) to compete with the test sample analyte (cytokine transcription factor) for binding with a limited amount of antibody. The amount of cytokine transcription factor in the test sample is inversely proportional to the amount of standard that becomes bound to the antibodies. To facilitate determining the amount of standard that becomes bound, the antibodies generally are solubilised before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilised on a solid support, and thereafter a second antibody binds to the analyte, thus forming an insoluble three part complex. David, et al., U.S. Pat No. 4,376,110. The second antibody may itself be labelled with a detectable moiety (direct sandwich assays) or may be measured using an anti-immunoglobulin antibody that is labelled with a detectable moiety (indirect sandwich assay). For example, one type of sandwich assay is an ELISA assay, in which case the detectable moiety is an enzyme.

The invention will now be further described by way of reference only to the following non-limiting examples. It should be understood, however, that the examples following are illustrative only, and should not be taken in any way as a restriction on the generality of the invention described above. In particular, while the invention is described in detail in relation to the detection of GATA-3 from HDM exposed PMBC, it will be clearly understood that the findings herein are not limited to these specific allergens or cytokines.

### Example 1 Specific Expression of GATA-3 mRNA in Response to Allergen

Blood samples were obtained from five atopic adult volunteers, who were selected on the basis of positive serum IgE responses to House Dust Mite (HDM), together with samples from five non-atopic controls who were tested for the presence of HDM-specific IgE in serum and were all negative. The presence of IgE to HDM was defined by the RAST (CAP) system (Pharmacia, Australia), and the positive volunteers in this study displayed RAST (CAP) scores ≥2. The allergy status of the test and control subjects is summarised in Table 1.

**Table 1**

| Allergy status of test subjects | | | | | | |
|---|---|---|---|---|---|---|
| **HDM IgE Positive** | | | | **HDM IgE negative** | | |
| **Subject** | **RAST** | **SPT** | | **Subject** | **RAST** | **SPT** |
| RG | 3 | 8 | | CM | 0 | 0 |
| BH | 4 | ND | | AC | 0 | 0 |
| SP | 3 | 9.5 | | KC | 0 | 0 |
| JP | 6 | ND | | TM | 0 | 4.5 |
| MD | 3 | ND | | AA | 0 | 0 |

Freshly isolated peripheral blood mononuclear cells (PBMC) were resuspended at 1x10⁶ cells/ml and 1ml of the cell suspension was cultured for 24 hours at 37°C, 5% CO₂ in round bottom tubes in serum-free medium AIM-V⁴ (Life Technologies, Mulgrave, Australia) supplemented with 4x10⁻⁵ 2-mercaptoethanol, with or without the addition of 10µg/ml of whole extract of HDM (*Dermatophagoides pteronyssinus,* CSL Limited, Parkville, Australia), or with the polyclonal mitogen soluble monoclonal anti-CD3 antibody (1:100 culture supernatant from OKT3 cell line, ATCC, USA) in combinatin with 20U/ml recombinant human IL-2. After culture, the cells were centrifuged, the supernatants were collected and stored at -20ºC for future analysis of cytokine content, and the cell pellet was used immediately for total RNA extraction.

Messenger RNA encoding the transcription factors GATA-3 and c-Maf was assayed by reverse transcriptase PCR (RT-PCR). Messenger RNA encoding beta-actin was assayed as a control for non-specific protein synthesis.

PCR products were generated and detected generally as previously described (Macaubas et al., 1999; Yabuhara et al., 1997). The GATA-3 primer sequences employed, which generated a 454 bp product, were
5' GAC GAG AAA GAG TGC CTC AAG 3' and
5' TCC AGA GTG TGG TTG TGG TG 3'.

The C-Maf primers, which generated a 479 bp product, were:
5' ACC TTC CAC AAT CAA GCC 3' and
5' GTA ACC CAT TCT GGT ATC TTT G 3'.

The annealing temperature for both GATA-3 and c-Maf primers was 57ºC, and the samples were amplified for 30 cycles.

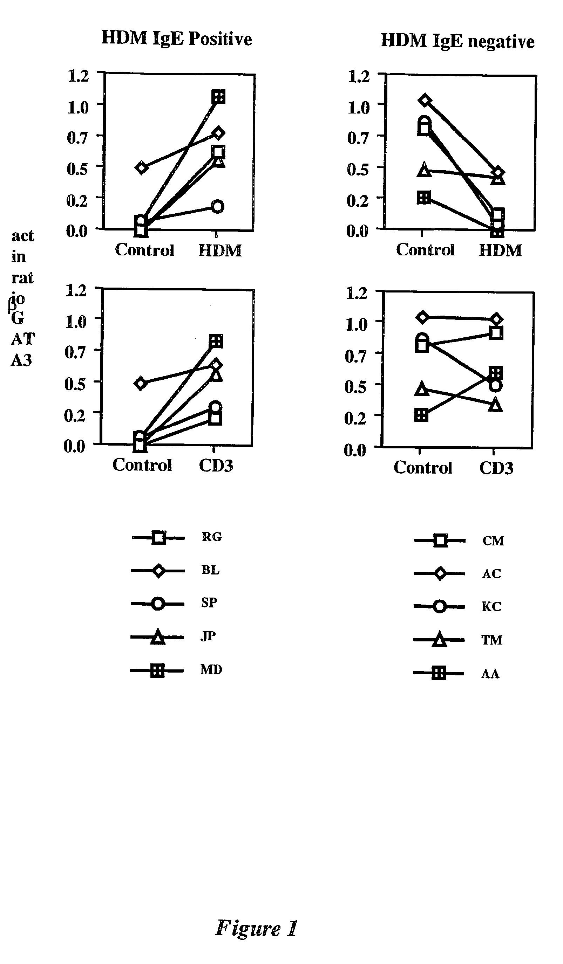

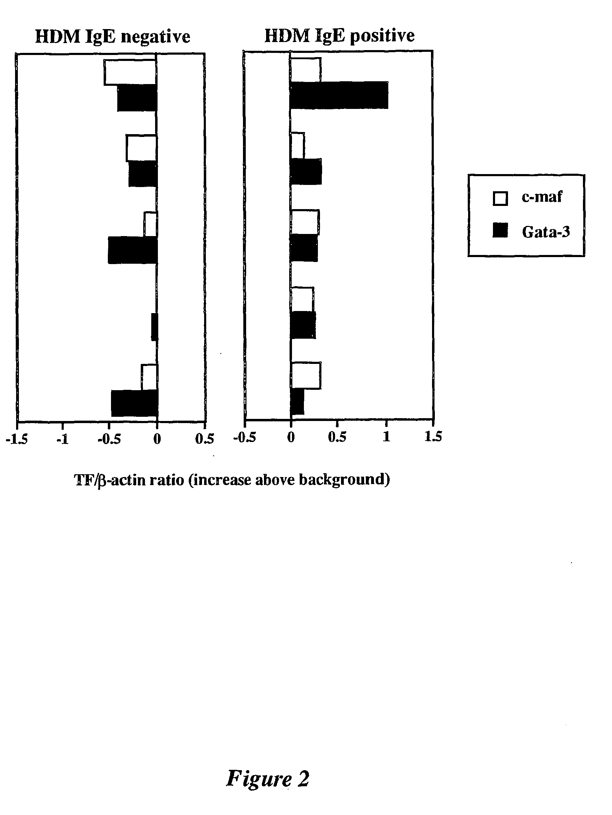

The PCR products were detected by slot-blot, employing biotinylated probes for β-actin, GATA-3 and c-Maf which were synthetised by PCR, as described previously (Macaubas et al., 1999; Yabuhara et al., 1997), using the same primers as above. The results, shown in Figure 1, are expressed as the ratio of transcription factor mRNA to beta-actin mRNA for each subject. It is evident that in each of the atopic subjects this ratio increased in response to HDM or to anti-CD3 antibody, whereas in the control subjects the ratio decreased in response to HDM; the response of these subjects to anti-CD3 antibody was variable, with two of the five showing a decrease, one no change, and two showing an increase. Figure 2 contrasts HDM-induced production of GATA-3 and c-maf in HDM-stimulated pbmc from HDM-sensitive atopics and non atopics. It is evident that the reciprocal patterns of GATA-3 expression which distinguish atopics and non atopics are to a significant extent mirrored in respective patterns of c-maf expression.

Thus we have demonstrated that when human PBMC are stimulated *in vitro* with a specific allergen, House Dust Mite allergen (HDM), the expression of mRNA encoding the transcription factor known as GATA-3 is increased in atopic subjects who are positive responders to HDM as measured by skin prick test reactivity and the presence of serum IgE antibody specific for HDM, and a similar pattern is seen with c-maf. In contrast, the expression of this mRNA is decreased in non-atopic subjects who are skin prick test negative/IgE negative. A similar increase in GATA-3 mRNA expression was observed in some atopic subjects when their PBMC were cultured with a polyclonal mitogen.

### Example 2 Specific Immunodiagnostic Methods

(i) PBMC from patients complaining of allergy symptoms are cultured overnight in separate aliquots with individual allergens to determine response status by measuring allergen-induced changes in levels of mRNA specific for different transcription factors, in cell lysates;
(ii) PBMC from cancer patients undergoing tumour-specific immunotherapy are cultured with tumour antigen, and the resulting pattern of transcription factor expression determined to monitor underlying changes in host anti-tumour immune responses;
(iii)PMBC from recipients of kidney transplant are cultured with kidney antigen, and the resulting pattern of transcription factor expression determined in order to identify the covert onset of graft rejection.

### Example 3 Confirmation by Real-Time PCR

After we have completed the analyses of the samples using the standard RT-PCR/slot blot procedure, PCR analysis by the real-time method (TaqMan) was used to confirm the results obtained from Example 1. The cDNA generated from subjects samples and polarised cell lines as described in Example 1 were used.

18S rRNA, GATA-3 and IL-12RB2 DNA standards were generated by PCR amplification of cDNA obtained from PHA stimulated (24h) PBMCs, using the same primers as for the real time reaction. PCR products were cloned into the vector pCR 2.1 using the original TA Cloning kit (Invitrogen, Carlsbad, CA), and *E. coli* competent cells (Invitrogen) were transformed with such vectors. The sequence identity of each DNA insert was confirmed by cycle sequencing. Serial 10-fold dilutions from each purified plasmid preparations were used as standards. β-actin standard was used as a serial 1:2 dilution of PHA stimulated cDNA. The linear range of the PCR for 18S rRNA, GATA-3 and IL-12RB2 was from 1 copy to 10 copies of plasmid, and for the β-actin, from neat to 1:128 dilution. For β-actin reactions the correlation coefficients were 0.98 and 0.99. For GATA-3 it was between 0.93 and 0.98. For IL-12 RB2 was 0.81 and for 18S rRNA, 0.98 PCR reaction and quantitation PCR premixes were prepared using Platinum^{™} Quantitative PCR Super Mix-UDG (Life Technologies), MgCL2 at 5.5mM (final concentration), and optimised concentration of primers and probe (Table 1). 18S rRNA was used as a commercially available premix at 1:20 final dilution (Applied Biosystems). Standard TaqMan conditions were used, except 2 minutes instead of 10 min for Taq polymerase activation.

For each assay the baseline was determined manually, and the threshold cycle (Ct) for each well was compared to the standard curve of the standards serial dilutions. The ratio between the relative amount of target genes and housekeeping (18S rRNA for cell lines and β-actin for subject samples) was calculated and the resulting figures expressed as "Taqman units".

GATA-3 RT-PCR primers and dual-labelled fluorogenic probe were designed using the program Primer Express (Applied Biosystems). The primer pair was designed to span an exon/intron boundary, and amplification of RNA samples not reversed transcribed was not observed. Amplification of these samples for 18S rRNA (which amplifies genomic DNA) showed amplification, demonstrating the presence of contaminating genomic DNA. Primers and probes for 18S rRNA were purchased directly from Applied Biosystems and used as per manufacture's instructions. Analyses of RNA samples not transcribed showed that the Ct values for these sample were approximately 10 cycles lower than levels detected in transcribed samples.

β-actin was also designed across exon/intron boundaries. IL-12RB2 primers and probe sequences were kindly provided by Dr M. Jenmaln. Analyses of RNA samples not transcribed showed that Ct values for these samples were around 8 cycles lower than transcribed samples. Probes were synthetised by Applied Biosystems (Table 1).

Analyses of cDNAs from 5 of the HDM-IgE positive and 4 of the HDM-IgE negative subjects from Example 1 by real time PCR showed a similar pattern to the results obtained with slot blot, ie, increased expression of GATA-3 after HDM stimulation in the HDM-IgE positive group and decrease in the HDM-IgE negative group (Table 2). It is not feasible to make direct correlations between values obtained through the slot blot experiment and the ones using real time PCR, due to the more extensive linear range of the TaqMan method. Nevertheless, the trend observed with the two methods are very similar. It is noteworthy that the "outlier" HDM-IgE positive subject who showed down regulation to HDM in the slot blot (positive subject 5 in Table 2) gave qualitatively similar results using the TaqMan. Replicate samples (cultured one year apart) from one subject (1/1a) also gave very similar results.

**Table 2**

| HDM-specific GATA-3 mRNA levels using slot blot and real time PCR (TaqMan). The results are expressed as GATA-3/β-actin ratio. | | | | | | | |
|---|---|---|---|---|---|---|---|
| HDM-IgE | | Slot blot | | | TaqMan | | |
| Negative | subject | control | HDM | HDM(d) | control | HDM | HDM(d) |
| | 1 | 1.05 | 0.47 | -0.58 | 105.74 | 2.01 | -103.73 |
| | 1a | nt | nt | | 103.44 | 21.01 | -82.43 |
| | 2 | 0.26 | 0 | -0.26 | 22.21 | 0 | -22.21 |
| | 3 | 0.87 | 0.06 | -0.81 | 37.04 | 0 | -37.04 |
| | 4 | 0.51 | 0.53 | 0.02 | 26.25 | 27.67 | -1.42 |
| | | | | | | | |
| Positive | 1 | 0 | 0.57 | 0.57 | 0 | 31.29 | 31.29 |
| | 2 | 0.46 | 0.82 | 0.35 | 0 | 368.8 | 368.8 |
| | 3 | 0.06 | 1.08 | 1.02 | 0 | 80.73 | 80.73 |
| | 4 | 0.29 | 0.62 | 0.33 | 21.33 | 591.58 | 570.25 |
| | 5 | 0.47 | 0.05 | -0.42 | 17.19 | 0 | -17.19 |

| | | | | | | | |
|---|---|---|---|---|---|---|---|
| (d): delta (the difference between GATA-3 stimulated and unstimulated levels); nt: not tested | | | | | | | |

It will be apparent to the person skilled in the art that while the invention has been described in some detail for the purposes of clarity and understanding, various modifications and alterations to the embodiments and methods described herein may be made without departing from the scope of the inventive concept disclosed in this specification.

References cited herein are listed on the following pages, and are incorporated herein by this reference.

### REFERENCES

1. Murphy, K.M., W. Ouyang, J.D. Farrar, J. Yang, S. Ranganath, H. Asnagli, M. Afkarian, and T.L. Murphy. 2000. Signalling and transcription in T helper development. Annu Rev Immunol. 18:451-494.
2. Zheng, W.-P., and R.A. Flavell. 1997. The transcription factor GATA-3 is necessary and sufficient for Th2 cytokine gene expression in CD4 T cells. Cell. 89:587-96.
3. Ho, I., D. Lo, and L. Glimcher. 1998. c-maf promotes T helper cell type 2 (Th2) and attenuates Th1 differentiation by both interleukin 4-dependent and - independent mechanisms. J Exp Med. 188:1859-66.
4. Zhang, D., L. Cohn, P. Ray, K. Bottomly, and A. Ray. 1997. Transcription factor GATA-3 is differentially expressed in murine Th1 and Th2 cells and controls Th2-specific expression of the interleukin-5 gene. J Biol Chem. 272:21597-21603.
5. Miaw, S.C., A. Choi, E. Yu, H. Kishikawa, and I.C. Ho. 2000. ROG, repressor of GATA, regulates the expression of cytokine genes. Immunity. 12:323-33.
6. Bird, J.J., D.R. Brown, A.C. Mullen, N.H. Moskowitz, M.A. Mahowald, J.R. Sider, T.F. Gajewski, C.-R. Wang, and S.L. Reiner. 1998. Helper T cell differentiation is controlled by the cell cycle. Immunity. 9:229-237.
7. Romagnani, S. 1991. Human TH1 and TH2 subsets: doubt no more. Immunol Today. 12:256-7.
8. Borish, L., and L. Rosenwasser. 1997. Th1/Th2 lymphocytes: doubt some more. J†Allergy Clin Immunol. 99:161-164.
9. Macaubas C, Sly PD, Burton P, Tiller K, Yabuhara A, Holt BJ, Smallacombe TB, Kendall G, Jenmalm M, Holt PG. Regulation of Th-cell responses to inhalant allergen during early childhood. Clin Exp Allergy 1999; 29:1223-1231.
10. Yabuhara A, Macaubas C, Prescott SL, Venaille T, Holt BJ, Habre W, Sly PD, Holt PG. Th-2-polarised immunological memory to inhalant allergens in atopics is established during infancy and early childhood. Clin Exp Allergy 1997; 27:1261-1269.

## Claims

1. A method of assessing an immunological reaction in a test subject suspected to be manifesting an abnormal immunological reaction, comprising the steps of
substantially isolating leukocytes from a biological sample obtained from a test subject and from a control subject;
stimulating said leukocytes by exposure to a stimulus;
measuring the level of expression of a transcription factor in said leukocytes; and
comparing the levels of expression in stimulated leukocytes in the test subjects and in the control subjects and comparing the levels of expression to unstimulated leukocytes from these subjects,
wherein significantly different expression in the test subject compared to that in the control subject is indicative of an abnormal immunological reaction.

2. A method according to claim 1, wherein the test subject shows higher expression than the control.

3. A method according to claim 1, wherein the test subject shows lower expression than the control.

4. A method according to any one of claims 1 to 3, wherein the stimulus is selected from the group consisting of allergen, heterologous or self antigen, mitogen and hormone.

5. A method according to claim 4, wherein said mitogen is concanavalin A or phytohaemagglutinin.

6. A method according to claim 5, wherein said allergen is House Dust Mite.

7. A method according to any one of claims 1 to 6, wherein said leukocytes are isolated from a biological sample.

8. A method according to claim 7, wherein said biological sample includes tissue or fluid isolated from bone marrow, plasma, serum, spinal fluid, lymph fluid, the external sections of the skin, respiratory, intestinal, and genitourinary tracts, tears, saliva, milk, blood; both whole blood and anticoagulated whole blood, blood cells, tumours, organs, or in vivo cell culture constituents.

9. A method according to claim 7 or 8, wherein said biological sample is blood, lymph fluid or a blood component.

10. A method according to any one of claims 7 to 9, wherein said biological sample is peripheral blood.

11. A method according to any one of claims 1 to 10, wherein said leukocytes are PBMC or unfractioned peripheral blood leukocytes or subfractions thereof.

12. A method according to any one of claims 1 to 11, wherein said transcription factor is GATA-3, t-bet, NFAT, NFKκB, ROG, STAT4, STAT6, IRF-1, or c-maf .

13. A method according to any one of claims 1 to 12, wherein transcription factor expression is effected by detection and quantitation of specific mRNA encoding the transcription factor, by polymerase chain reaction (PCR), or via specific DNA arrays utilising microchip technology.

14. A method according to any one of claims 1 to 12, wherein expression of transcription factor is effected by measurement of the transcription factor protein by ELISA, proteomic arrays, or intracellular staining as detected by flow cytometry.

15. A method according to any one of claims 1 to 14, for the detection of immunologically-mediated conditions.

16. A method according to claim 14, wherein said immunologically-mediated condition is allergy, autoimmunity, graft rejection or immunodeficiency.

17. A method according to any one of claims 1 to 14, for the assessment of immune reactivity to antigens.

18. A method according to claim 17, wherein said antigens are associated with cancer, viral infection, AIDS or tissue transplantation.

19. A method according to any one of claims 1 to 14, for monitoring response of patients to immunotherapy or immunosuppression.

20. A method according to any one of claims 1 to 14, for the screening of diseases.

21. A method according to claim 20, wherein said diseases are tuberculosis, rheumatoid arthritis or type 1 diabetes.

22. A method according to any one of claims 1 to 14, for the assessment of potential disease susceptibility in a subject.

23. A method according to claim 22, wherein said subject is at high risk of autoimmunity, atopy or asthma.

24. A method according to any one of claims 1 to 14, for the routine assessment of immune competence following polyclonal stimulation.

## Patentansprüche

1. Verfahren zur Feststellung einer immunologischen Reaktion bei einem Testsubjekt, von dem vermutet wird, dass es eine anomale immunologische Reaktion zeigt, wobei das Verfahren die folgenden Stufen umfasst:
im Wesentlichen Isolieren von Leukozyten aus einer biologischen Probe, die von einem Testsubjekt und von einem Kontrollsubjekt erhalten wird;
Stimulieren der Leukozyten durch Einwirken eines Stimulus;
Ermitteln des Expressionsgrades eines Transkriptionsfaktors in den Leukozyten und
Vergleichen der Expressionsgrade in stimulierten Leukozyten in den Testsubjekten und in den Kontrollsubjekten und Vergleichen der Expressionsgrade mit nicht stimulierten Leukozyten von diesen Subjekten,
wobei eine signifikant unterschiedliche Expression in dem Testsubjekt im Vergleich zu der im Kontrollsubjekt eine anomale immunologische Reaktion anzeigt.

2. Verfahren nach Anspruch 1, wobei das Testsubjekt eine höhere Expression als die Kontrolle zeigt.

3. Verfahren nach Anspruch 1, wobei das Testsubjekt eine niedrigere Expression als die Kontrolle zeigt.

4. Verfahren nach einem der Ansprüche 1 bis 3, wobei der Stimulus aus der Gruppe von einem Allergen, einem heterologen oder eigenen Antigen, Mitogen und einem Hormon ausgewählt ist.

5. Verfahren nach Anspruch 4, wobei das Mitogen Concanavalin A oder Phytohämagglutinin ist.

6. Verfahren nach Anspruch 5, wobei das Allergen die Hausstaubmilbe ist.

7. Verfahren nach einem der Ansprüche 1 bis 6, wobei die Leukozyten aus einer biologischen Probe isoliert werden.

8. Verfahren nach Anspruch 7, wobei die biologische Probe Gewebe oder Flüssigkeit, die aus Knochenmark, Plasma, Serum, Liquor, Lymphflüssigkeit, den äußeren Bereichen der Haut, dem Atemwege-, Intestinal- und Urogenitaltrakt, Tränen, Speichel, Milch, Blut isoliert wurden; sowohl Vollblut als auch antikoaguliertes Vollblut, Blutzellen, Tumore, Organe oder in-vivo-Zellkulturbestandteile umfasst.

9. Verfahren nach Anspruch 7 oder 8, wobei die biologische Probe Blut, Lymphflüssigkeit oder eine Blutkomponente ist.

10. Verfahren nach einem der Ansprüche 7 bis 9, wobei die biologische Probe peripheres Blut ist.

11. Verfahren nach einem der Ansprüche 1 bis 10, wobei die Leukozyten PBMC oder nicht fraktionierte Leukozyten von peripherem Blut oder Subfraktionen derselben sind.

12. Verfahren nach einem der Ansprüche 1 bis 11, wobei der Transkriptionsfaktor GATA-3, t-bet, NFAT, NFKκB, ROG, STAT4, STAT6, IRF-1 oder c-maf ist.

13. Verfahren nach einem der Ansprüche 1 bis 12, wobei die Transkriptionsfaktorexpression durch Detektion und quantitative Bestimmung von spezifischer mRNA mit Codierung für den Transkriptionsfaktor, durch Polymerasekettenreaktion (PCR) oder über spezifische DNA-Arrays unter Verwendung von Mikrochiptechnologie durchgeführt wird.

14. Verfahren nach einem der Ansprüche 1 bis 12, wobei die Expression eines Transkriptionsfaktors durch Ermittlung des Transkriptionsfaktorproteins durch ELISA, Proteomarrays oder intrazelluläre Anfärbung gemäß Detektion durch Durchflusszytometrie durchgeführt wird.

15. Verfahren nach einem der Ansprüche 1 bis 14 zur Detektion von immunologisch vermittelten Zuständen.

16. Verfahren nach Anspruch 14, wobei der immunologisch vermittelte Zustand eine Allergie, Autoimmunität, Transplantatabstoßung oder Immunschwäche ist.

17. Verfahren nach einem der Ansprüche 1 bis 14 zur Feststellung einer Immunreaktivität gegenüber Antigenen.

18. Verfahren nach Anspruch 17, wobei die Antigene mit Krebs, einer Virusinfektion, AIDS oder einer Gewebetransplantation in Verbindung stehen.

19. Verfahren nach einem der Ansprüche 1 bis 14 zur Überwachung der Antwort von Patienten auf eine Immuntherapie oder Immunsuppression.

20. Verfahren nach einem der Ansprüche 1 bis 14 zum Screening von Erkrankungen.

21. Verfahren nach Anspruch 20, wobei die Erkrankungen Tuberkulose, rheumatoide Arthritis oder Typ-1-Diabetes sind.

22. Verfahren nach einem der Ansprüche 1 bis 14 zur Feststellung einer möglichen Erkrankungsempfindlichkeit bei einem Subjekt.

23. Verfahren nach Anspruch 22, wobei das Subjekt ein hohes Risiko für Autoimmunität, Atopie oder Asthma aufweist.

24. Verfahren nach einem der Ansprüche 1 bis 14 zur Routinefeststellung von Immunkompetenz nach einer polyklonalen Stimulation.

## Revendications

1. Procédé d'évaluation d'une réaction immunologique chez un sujet à tester soupçonné de manifester une réaction immunologique anormale, comprenant les étapes consistant
à isoler essentiellement les leucocytes d'un échantillon biologique obtenu auprès d'un sujet à tester et auprès d'un sujet témoin ;
à stimuler lesdits leucocytes par exposition à un stimulus ;
à mesurer le taux d'expression d'un facteur de transcription dans lesdits leucocytes ; et
à comparer les taux d'expression dans les leucocytes stimulés chez les sujets à tester et chez les sujets témoins et à comparer les taux d'expression dans les leucocytes non stimulés de ces sujets,
dans lequel une expression significativement différente chez le sujet à tester par rapport à celle chez le sujet témoin est indicative d'une réaction immunologique anormale.

2. Procédé selon la revendication 1, dans lequel le sujet à tester présente une expression plus élevée que le témoin.

3. Procédé selon la revendication 1, dans lequel le sujet à tester présente une expression plus basse que le témoin.

4. Procédé selon l'une quelconque des revendications 1 à 3, dans lequel le stimulus est choisi dans le groupe constitué par un allergène, un antigène hétérologue ou un auto-antigène, un mitogène et une hormone.

5. Procédé selon la revendication 4, dans lequel ledit mitogène est la concanavaline A ou la phytohémagglutinine.

6. Procédé selon la revendication 5, dans lequel ledit allergène est un acarien dermatophagoïde.

7. Procédé selon l'une quelconque des revendications 1 à 6, dans lequel lesdits leucocytes sont isolés à partir d'un échantillon biologique.

8. Procédé selon la revendication 7, dans lequel ledit échantillon biologique inclut un tissu ou un fluide isolé de moelle osseuse, de plasma, de sérum, de fluide spinal, de fluide lymphatique, des sections externes de la peau, des tractus respiratoire, intestinal et génito-urinaire, de larmes, de salive, de lait, de sang ; à la fois de sang entier et de sang entier anticoagulé, de globules sanguins, de tumeurs, d'organes, ou de constituants de culture cellulaire *in vivo*.

9. Procédé selon la revendication 7 ou 8, dans lequel ledit échantillon biologique est du sang, du fluide lymphatique ou un constituant sanguin.

10. Procédé selon l'une quelconque des revendications 7 à 9, dans lequel ledit échantillon biologique est du sang périphérique.

11. Procédé selon l'une quelconque des revendications 1 à 10, dans lequel lesdits leucocytes sont des PBMC ou des leucocytes de sang périphérique non fractionnés ou des sous-fractions de ceux-ci.

12. Procédé selon l'une quelconque des revendications 1 à 11, dans lequel ledit facteur de transcription est GATA-3, t-bet, NFAT, NFKκB, ROG, STAT4, STAT6, IRF-1 ou c-maf.

13. Procédé selon l'une quelconque des revendications 1 à 12, dans lequel l'expression du facteur de transcription est effectuée par détection et quantification d'ARNm spécifique codant le facteur de transcription, par réaction en chaîne par polymérase (PCR), ou par des puces d'ADN spécifiques en utilisant une technique à micropuce.

14. Procédé selon l'une quelconque des revendications 1 à 12, dans lequel l'expression du facteur de transcription est effectuée par dosage de la protéine du facteur de transcription par ELISA, des puces protéomiques, ou coloration intracellulaire telle que détectée par cytométrie de flux.

15. Procédé selon l'une quelconque des revendications 1 à 14, pour la détection d'affections d'origine immunologique.

16. Procédé selon la revendication 14, dans lequel ladite affection d'origine immunologique est une allergie, une auto-immunité, un rejet de greffon ou une immunodéficience.

17. Procédé selon l'une quelconque des revendications 1 à 14, pour l'évaluation d'une réactivité immunitaire à des antigènes.

18. Procédé selon la revendication 17, dans lequel lesdits antigènes sont associés à un cancer, à une infection virale, au SIDA ou à une transplantation de tissu.

19. Procédé selon l'une quelconque des revendications 1 à 14, pour suivre la réponse de patients à une immunothérapie ou une immunosuppression.

20. Procédé selon l'une quelconque des revendications 1 à 14, pour le dépistage de maladies.

21. Procédé selon la revendication 20, dans lequel lesdites maladies sont la tuberculose, la polyarthrite rhumatoïde ou le diabète de type 1.

22. Procédé selon l'une quelconque des revendications 1 à 14, pour l'évaluation d'une sensibilité potentielle à une maladie chez un sujet.

23. Procédé selon la revendication 22, dans lequel ledit sujet présente un risque élevé d'autoimmunité, d'atopie ou d'asthme.

24. Procédé selon l'une quelconque des revendications 1 à 14, pour l'évaluation régulière d'une compétence immunitaire après stimulation polyclonale.
